# EUROPEAN PATENT APPLICATION

(11) **EP 1 932 533 A1**
(43) Date of publication of application: **18.06.2008**
(21) Application number: 06797352.9
(22) Date of filing: 04.09.2006
(51) Int. Cl.: A61K 36/899, A23L 1/305, A61K 38/00, A61P 3/04, A61P 3/06, A61P 3/10

(54) **COMPOSITION FOR AMELIORATION OF BODY LIPID**

(30) Priority: 05.09.2005 JP 2005256287; 31.03.2006 JP 2006100613
(71) Applicant: Tsuno Food Industrial Co., Ltd., Ito-gun Wakayama 649-7194 (JP)
(72) Inventor: TSUNO, Takuo, Ito-gun, Wakayama 649-7194 (JP); KARIYA, Tetsuya, Ito-gun, Wakayama 649-7194 (JP); KAKIUCHI, Makoto, Ito-gun, Wakayama 649-7194 (JP); MINAMI, Yumi, Ito-gun, Wakayama 649-7194 (JP); KOYAMA, Atsushi, Ito-gun, Wakayama 649-7194 (JP); ISHIMOTO, Takako, Ito-gun, Wakayama 649-7194 (JP)
(74) Representative: Weller, Wolfgang
(86) International application number: PCT/JP2006/317427
(87) International publication number: WO 2007/029631

(57) **Abstract**

An object of the present invention is to obtain a composition, which is excellent in amelioration of a lipid metabolism disorder and has a preventive or ameliorating effect on hyperlipemia, obesity or type II diabetes induced by the lipid metabolism disorder, and is also economical and safe with respect to allergy, by extracting a rice bran extract containing a rice bran protein, followed by separation.

Disclosed is a composition for amelioration of a lipid metabolism disorder, containing a rice bran protein.

## Description

### TECHNICAL FIELD

The present invention relates to a composition containing a rice bran protein for amelioration of a lipid metabolism disorder. More particularly, the present invention relates to a novel composition for amelioration of a body lipid metabolism disorder, which contains a rice bran protein obtained from rice bran and reduces a body lipid such as cholesterol and neutral fat in the blood, liver, or the like.

### BACKGROUND ART

Rice bran is an important plant resource rich in lipid, protein, dietary fiber, vitamins and minerals. In Japan, rice bran is generated in an amount of about 900,000 tons per year, and rice oil produced from the rice bran is an important domestic vegetable oil.

The residue after extracting rice oil from the rice bran is called defatted rice bran. The defatted rice bran contains the above-mentioned active constituents except for lipid. Various attempts have long been made on exploitation of the defatted rice bran as food materials, culture media, food additives, medical supplies and cosmetic materials. Currently, inositol and phytic acid are industrially produced from the defatted rice bran on a mass scale as raw materials for medicines and cosmetics and food additives. However, production of a protein and vitamins has yet to be practiced out of the defatted rice bran.

On the other hand, as the westernization of food proceeds in recent years, there emerges a strong tendency that the concentrations of cholesterol and neutral fat are increasing in the blood and liver. The increase in the concentrations of cholesterol and neutral fat in the blood and others is said to be a cause for various diseases including arterial sclerosis. Various components have been suggested in the pharmaceutical and food sectors aiming at reducing cholesterol and neutral fat, and among others, proteins have been drawing attention particularly in the food sector as a cholesterol-reducing component together with sterol. It has been known that a soybean protein and dried egg albumen after a dry heat treatment serve as the cholesterol-reducing component (see, for example, Patent Documents 1 and 2). A neutral fat reducing composition has also been devised, which contains a low phytic acid soybean protein containing 7S globulin in an amount of not less than 59%, which is fractionated from a soybean protein, as an active constituent (see Patent Document 3).

However, these compositions have not been widely used since the effect of reducing cholesterol and neutral fat they have is moderate and there is an allergic problem and so on. The practice of fractionation/purification to raise the effect of the compositions is highly likely to remove allergic causes, but it also raises the cost and entraps them into economic inferiority.
Patent Document 1: Japanese Patent Publication Laid-Open (JP-A) No. 2002-114694
Patent Document 2: JP-A No. 2000-16943
Patent Document 3: WO2002/026243

### DISCLOSURE OF THE INVENTION

### PROBLEMS TO BE SOLVED BY THE INVENTION

The object of the present invention is to provide a composition, which is excellent in amelioration of a lipid metabolism disorder and has a preventive or ameliorating effect on hyperlipemia, obesity or type II diabetes induced by the lipid metabolism disorder, and is also economical and safe with respect to allergy, by extracting a rice bran component containing a rice bran protein, which is an unused resource, followed by separation.

### MEANS FOR SOLVING THE PROBLEMS

In order to solve the above problems, the present inventors have intensively studied and performed an animal test using rats by comparing an extract containing a rice bran protein obtained from defatted rice bran with casein and a soybean protein. As a result, they have found that the extract containing the rice bran protein is excellent in the effect of reducing cholesterol and neutral fat in the blood or liver. Based on this finding, the present inventors have further studied, and thus the present invention has been completed.

That is, the present invention pertains to:
(1) a composition for amelioration of a lipid metabolism disorder, which comprises a rice bran protein,
(2) the composition according to (1), wherein the rice bran protein is a protein contained in rice bran extract,
(3) the composition according to (2), wherein the amount of the rice bran protein relative to the rice bran extract is not less than 50% by mass,
(4) the composition according to any one of (1) to (3), wherein the lipid metabolism disorder is caused by at least one disease selected from hyperlipemia, obesity and type II diabetes,
(5) the composition according to any one of (1) to (4), which is a medicine,
(6) the composition according to any one of (1) to (5), which is a food article or a food material,
(7) a method for amelioration of a lipid metabolism disorder, which comprises administering a composition containing a rice bran protein to a mammal including a human,
(8) the method according to (7), wherein the rice bran protein is a protein contained in rice bran extract,
(9) the method according to (8), wherein the amount of the rice bran protein relative to the rice bran extract is not less than 50% by mass,
(10)the method according to any one of (7) to (9), wherein the lipid metabolism disorder is caused by at least one disease selected from hyperlipemia, obesity and type II diabetes,
(11)use of a rice bran protein for producing a medicine, a food article or a food material for amelioration of a lipid metabolism disorder,
(12)the use according to (11), wherein the rice brain protein is a protein contained in a rice bran extract,
(13)the use according to (12), wherein the amount of the rice bran protein relative to the rice bran extract is not less than 50% by mass,
(14)the use according to any one of (11) to (13), wherein the lipid metabolism disorder is caused by at least one disease selected from hyperlipemia, obesity and type II diabetes,
(15)a food article containing a rice bran protein, which is labeled that the food article is used for prevention or amelioration of a lipid metabolism disorder, and
(16)a food article containing a rice bran protein, which is labeled that the food article is used for prevention or amelioration of hyperlipemia, obesity or type II diabetes.

### EFFECT OF THE INVENTION

The composition of the present invention can suppress the increase of neutral fat and total cholesterol in the blood and liver, which may be derived from a lipid metabolism disorder or high fat diet, age or lack of exercise.

The composition of the present invention can prevent, treat or ameliorate hyperlipemia since it can ameliorate the lipid metabolism disorder. Furthermore, the composition can prevent cerebral arteriopathy such as cerebral infarction, and coronary artery diseases such as angina pectoris and cardiac infarction from being triggered since the prevention or the like of hyperlipemia serves to prevent arterial sclerosis in the heart and brain derived from hyperlipemia.

The composition of the present invention can prevent or ameliorate obesity, particularly visceral fat accumulation-type obesity. Since the visceral fat accumulation-type obesity serves as a trigger for lifestyle-related diseases such as diabetes and hypertension, the prevention and amelioration of the visceral fat accumulation-type obesity can lead to the prevention, treatment or amelioration of lifestyle-related diseases. The composition of the present invention can also suppress the increase of leptin in the blood whose secretion increases and concentration rises by obesity. Since the concentration of leptin in the blood is correlated with blood pressure, the composition of the present invention that suppresses the increase in the concentration of leptin in the blood can suppress the blood pressure elevation as well.

The composition of the present invention can ameliorate insulin sensitivity since it can suppress the decline of adiponectin in the blood, whose secretion is reduced by high fat diet. Although insulin resistance and coronary artery diseases are triggered when the concentration of adiponectin in the blood declines, the composition of the present invention can further promote the secretion of adiponectin and therefore is useful for prevention, treatment or amelioration of type-II diabetes, particularly insulin resistant diabetes, and coronary artery diseases.

The composition of the present invention can increase the fecal volume and also shorten the intestinal passage time of food. As a result, food stays in the intestine for a shorter time and thus glucide and lipid are less absorbed in the intestine. This serves for prevention of obesity and for suppression of increase in blood sugar and secretion of insulin after meal, and can be useful for prevention and treatment of diabetes. In addition, by shortening the intestinal passage time of food, the increase of harmful bacteria can be suppressed in the intestine and thus colon cancer and other diseases can be prevented.

Since the rice bran protein used in the present invention has extremely low toxicity such as an allergic factor and is used safely as a food material, the composition of the present invention containing the rice bran protein is useful as a medicine, a food article or a food material.

### BEST MODE FOR CARRYING OUT THE INVENTION

The rice bran protein used in the present invention is a protein component contained in rice bran, and is obtained by extraction of rice bran with a proper solvent. Examples of the rice bran include those obtained during polishing hulled rice. The rice bran is not particularly limited, and includes defatted rice bran obtained after extracting an oil content from the rice bran, for example. The defatted rice bran without an oil content allows a protein to be extracted efficiently and is excellent in operability too. Furthermore, the protein prepared from the defatted rice bran contains no oil content, which enhances flavor and storage stability. From an economic point of view, and the like, the defatted rice bran is preferred.

The rice bran protein of the present invention is prepared from rice bran, and the extraction method can employ a common extraction method for proteins. For example, the extraction operation of rice bran in an aqueous solution or an organic solvent gives an extract of a protein in a high yield. Particularly, extraction using an aqueous solution, more preferably using an aqueous neutral or weak alkaline solution, gives a crude extract of the rice bran protein.

Examples of the solvent used in the present invention include an aqueous neutral or weak alkaline solution such as water (for example, purified water, distilled water, tap water, or the like), saline solution (for example, sodium chloride, and the like) and an alkaline solution which can be used for the preparation of a food material; an organic solvent used for the preparation of a food material (for example, ethanol, or the like); an appropriate combination of these solvents, and the like. More specifically, the solvent may be a dilute salt solution, a neutral or weak alkaline solution, or an aqueous neutral or weak alkaline solution containing about 5 to 90 vol% of ethanol, or the like, and the aqueous neutral or weak alkaline solution is preferably used. The pH of the aqueous neutral or weak alkaline solution is preferably from about 6.5 to 12, more preferably from about 6.5 to 11, still more preferably from about 7.0 to 11, further preferably from about 7.5 to 10, and most preferably from about 7.5 to 9. In the present invention, the rice bran protein can be efficiently extracted as long as the pH is within the above range. Examples of the alkali include sodium hydroxide, potassium hydroxide and the like. An aqueous solution is preferred as the above solution.

Any extraction method can be applied to obtaining a rice bran extract containing a rice bran protein from rice bran by using a solvent, as long as the method extract the rice bran protein efficiently. For example, the rice bran and a solvent are mixed to give a mixture solution. The mixing ratio of the rice bran and the solvent is not particularly limited, and the amount of the solvent is from about 5 to 50 parts by mass, preferably from about 5 to 20 parts by mass, and more preferably from about 8 to 15 parts by mass, based on 1 part by mass of the rice bran. When the rice bran protein is extracted in an aqueous neutral or weak alkaline solution, for example, the rice bran and the aqueous neutral or weak alkaline solution adjusted within the above pH values may be mixed in the above mixing ratio, or the rice bran and water are mixed first in the above mixing ratio, followed by adjusting the pH to the above value using an about 10 to 30% by mass of aqueous sodium hydrochloride solution or aqueous potassium hydrochloride solution. It is preferred to stir or shake the mixture of the rice bran and the solvent so as to uniformly mix the rice bran.

Then, the rice bran protein contained in the rice bran in the mixture solution is solubilized and extracted into the solution. The extraction temperature is preferably from about 0 to 50°C, and more preferably from about 0 to 40°C. The above extraction temperature includes room temperature, too. The extraction time is not particularly limited, and is usually from about 0.5 to 100 hours, preferably from about 0.5 to 24 hours, and more preferably from about 0.5 to 5 hours. Examples of the extraction method include a stationary method, a stirring method, a shaking method, a column elution method and the like, and a stirring method or a shaking method is preferable.

Next, the rice bran extract containing the rice bran protein solubilized in the mixture solution is separated from the rice bran. Examples of the method for separation of the rice bran extract include centrifugal, filtration, vacuum membrane filtration, pressure membrane filtration and stationary decantation methods, and a centrifugal method is preferred. The rice bran extract contains dietary fiber and glucide as well as the protein in the rice bran. Although the rice bran extract obtained in this manner is usable as it is as the rice bran extract containing the rice bran protein, it is preferred to increase the concentration of the protein by such methods as acid precipitation, alcohol precipitation, salt precipitation, membrane separation and chromatograph separation as desired.

The acid precipitation includes a method in which an inorganic acid such as hydrochloric acid or sulfuric acid, an organic acid such as trichloroacetic acid or sulfosalicylic acid, or the like is added to the extract containing the rice bran protein in order to adjust the pH of the extract to a weak acidity, and the rice bran protein to be precipitated is separated. It is preferred that the pH value after addition of the acid is from about 2.5 to 5.5. The alcohol precipitation includes a method in which ethanol or the like is added to the extract containing the rice bran protein until it accounts for about 50 to 70 vol% and allows the rice bran protein to be precipitated, and then the precipitate is separated. It is also allowed to use an organic solvent such as acetone in place of the alcohol in order to precipitate the rice bran protein. The salt precipitation includes a method in which ammonium sulfate is added to the extract containing the rice bran protein so as to precipitate the rice bran protein. Among these, the acid precipitation method is preferred from an economic viewpoint and the like.

The precipitate separated and obtained in this manner has high rice bran protein content, and is preferred as the rice bran extract containing the rice bran protein. Although the precipitate obtained by separation is usable as it is as the rice bran extract containing the rice bran protein, it is also possible to obtain a rice bran extract containing a higher concentration of the rice bran protein after washing the precipitate obtained by the separation with an alcohol, an aqueous acidic solution or an aqueous solution containing various salts to remove glucide and phytin. This can be stored under freezing and thawed when it is used as the rice bran extract containing the rice bran protein, or the obtained precipitate can be dried by a proper known method to make a powder, so as to increase the storage stability.

Examples of the method to separate the precipitate include a centrifugal method, a membrane filtration method, a filtration method using a filter aid, and the like. When the precipitate is separated by a centrifugal method, it is preferred to conduct centrifugal separation in a centrifugal separator for about one minute to one hour at a rotary speed of from about 100 to 30,000 rpm, preferably from 500 to 3,000 rpm. Industrially, it is preferred to conduct the separation of the precipitate by a batch or continuous centrifugal treatment using a screw decanter, a basket type centrifugal machine, a Sharples centrifugal machine, a disk type centrifugal machine or the like. When the precipitate is separated by a membrane filtration method, filtration can be conducted by the pressure difference using a cellulose membrane or an ultrafilter membrane. It is also possible to conduct the filtrating using a filter aid such as celite. Although the drying method can be exemplified by air drying, heated-air drying, drying under room temperature and reduced pressure, vacuum drying, freeze drying and spray drying, freeze drying or spray drying is preferred among others.

Since the supernatant after separating out the precipitate also contains the rice bran protein, it is included in the rice bran extract containing the rice bran protein of the present invention. It is preferred that the supernatant is also used after concentrated, fractionated/purified, dried or the like. The concentration or drying method is exemplified by the above-mentioned methods.

The membrane separation method is exemplified by a method of concentrating the rice bran protein contained in the extract with an ultrafilter membrane or the like. The chromatograph separation method is exemplified by a method of subjecting the extract containing the rice bran protein to molecular sieve chromatography or ion exchange column chromatography.

The rice bran protein used in the present invention includes all proteins contained in the rice bran extract. The content rate of the rice bran protein in the rice bran extract is not particularly limited, and the mass of the crude protein is preferably not less than about 50% by mass, and more preferably not less than about 60% by mass, by a Kjeldahl method based on the rice bran extract (dry mass).

In the present invention, the lipid metabolism disorder indicates the state where an abnormality is recognized in the balance of a body lipid component, and hyperlipemia and accumulation of cholesterol and neutral fat in the liver are exemplified. The hyperlipemia includes the state where the value of total cholesterol, LDL (low density lipoprotein) cholesterol, neutral fat (TG), free fatty acid or remnant-like lipoprotein-cholesterol (RLP-cholesterol), for example, is high in the blood, and the state where the value of HDL (high density lipoprotein) cholesterol is low in the blood. The lipid metabolism disorder also includes the state of decline in the concentration of blood adiponectin or increase in the concentration of blood leptin, β-lipoprotein, apoprotein, lipoprotein or the like. The lipid metabolism disorder is not restricted by a cause as long as an abnormality is recognized in the body lipid balance. It includes a case where the disorder is caused by high fat diet, or caused by age or lack of exercise even though the dietary habit is normal. The hyperlipemia mentioned above can exert a grave effect on the progress of arterial sclerosis. Arterial sclerosis is a state where a lumen of an artery wall is narrowed or occluded, which leads to the decline in blood flow and may cause various diseases such as transient cerebral ischemia attack, cerebral infarction, cardiac infarction and angina pectoris.

There also raises the relation between the lipid metabolism disorder and obesity. Obesity is a state where fat excessively accumulates in a body, insulin resistance is present, and type II diabetes or hypertension can be triggered. Obesity includes visceral fat accumulation-type obesity.

Accordingly, the composition containing the rice bran protein used in the present invention is usable as a medicine that prevents or treats hyperlipemia, obesity, type II diabetes or hypertension.

The hyperlipemia, obesity (particularly, visceral fat accumulation-type obesity), type II diabetes or hypertension can trigger the above-mentioned various diseases on their own, but a state where these diseases are duplicated, such as a metabolic syndrome, promotes arterial sclerosis and is likely to trigger fatal cardiac infarction, cerebral infarction or the like. Since the composition containing the rice bran protein used in the present invention is able to suppress any diseases described above, such as hyperlipemia, it is capable of suppress the metabolic syndrome.

The composition of the present invention can be applied to the lipid metabolism disorder suffered by a human being and other mammals (for example, monkey, cow, horse, pig, sheep, dog, cat, rat, mouse, chimpanzee, and the like).

The composition relating to the present invention is produced by mixing the rice bran protein, and can be used as a medicine, a food article or a food material. It is preferred to use the rice bran extract containing the rice bran protein as the rice bran protein. When the protein is used as a medicine, it is preferred to administer it in a form of a pharmaceutical composition containing the rice bran extract containing the rice bran protein and pharmaceutically acceptable additives for preparations. The pharmaceutical composition may have the form of oral solid preparations such as capsules, tablets (including coat tablets such as sugar coated tablets and enteric tablets, and multi-layered tablets), powders and granulates, may have the form of oral liquid preparations, or may have the form of parenteral preparations such as injections, intravenous drip solutions and suppositories. These preparations can be prepared according to a method known per se.

Examples of an additive for preparations which are usually used for solid preparations such as capsules, tablets, powders or granulates include, but are not limited thereto, excipients (for example, lactose, sucrose, glucose, starch, crystalline cellulose, and the like), binders (for example, starch paste solution, hydroxypropylcellulose solution, carmellose solution, gum arabic solution, gelatin solution, sodium alginate solution, and the like), disintegrators (for example, starch, sodium carmellose, calcium carbonate, and the like), lubricants (for example, magnesium stearate, talc, stearic acid, calcium stearate, and the like), surfactants (for example, polysorbate 80, polyoxyethylene hardened ricinus, and the like) and thickeners (for example, hydroxyethylcellulose, hydroxypropylcellulose, polyvinylalcohol, polyethylene glycol, and the like). The tablet or the granulate can be coated with a coating agent (for example, gelatin, sucrose, gum arabic, carnauba wax, cellulose acetate phthalate, methacrylic acid copolymer, hydroxypropylcellulose phthalate, carboxymethylethylcellulose, hydroxypropylmethylcellulose, or the like). The capsule may be a microcapsule, a soft capsule or the like as well as a hard capsule. The solid preparation can be prepared by a known method.

The liquid preparation can be mixed with saccharides (for example, sucrose, sorbitol, fructose, and the like), glycols (for example, polyethylene glycol, propylene glycol, and the like), dispersants or thickeners (for example, gelatin, gum arabic, hydroxyethylcellulose, hydroxypropylcellulose, polyvinylalcohol, polyethylene glycol, and the like), emulsifiers (for example, glycerin fatty acid ester, sucrose fatty acid ester, and the like), solubilizing agents (for example, gum arabic, polysorbate 80, and the like), pH adjustors (for example, citric acid, trisodium citrate, and the like) and antiseptics (for example, p-hydroxybenzoic acid ester, and the like).

Among these preparations which are suitable for parenteral administration, those for intravascular administration such as an injection solution and an intravascular drip solution can be prepared preferably using an aqueous medium with the same isotonicity as a body fluid. For example, an injection solution can be prepared as a solution, a suspension or a dispersion according to an ordinary method using an aqueous medium selected from distilled water for injection, a salt solution, a glucose solution and a mixture of a salt solution and a glucose solution, together with an appropriate auxiliary agent. The suppository for intestinal administration can be prepared using a carrier such as cacao oil, hydrogenated fat or hydrogenated carboxylic acid.

As the rice bran protein used for food articles, the rice bran extract containing the rice bran protein can be used preferably. Although the rice bran protein or the rice bran extract can be used as it is as the food article, it is usually preferred to make a form that contains the rice bran extract containing the rice bran protein and additives which are food-hygienically acceptable, or common food materials. The food material may constitute a form that uses the rice bran protein or the rice bran extract containing the rice bran protein as it is, or may constitute a form that contains food-hygienically acceptable additives.

The food article and the food material may take the form of solid compositions, together with food-hygienically acceptable additives, such as capsules, tablets (including a coated tablet such as a sugar coated tablet, a multi-layered tablet or a mouth disintegrating tablet), powders and granulates, or may take the form of liquid compositions. Examples of the additives include excipients (for example, lactose, dextrin, corn starch, crystalline cellulose, and the like), lubricants (for example, magnesium stearate, sucrose fatty acid ester, glycerin fatty acid ester, and the like), disintegrators (for example, carboxymethylcellulose calcium, anhydrous calcium hydrogen phosphate, calcium carbonate, and the like), binders (for example, starch paste solution, hydroxypropylcellulose solution, gum arabic solution, and the like), solubilizing agents (for example, gum arabic, polysorbate 80, and the like), sweeteners (for example, sugar, fructose, glucose liquid sugar, honey, aspartame, and the like), artificial colors (for example, β-carotene, edible tar color, riboflavin, and the like), preservatives (for example, sorbic acid, methyl paraoxybenzoate, sodium sulfite, and the like), thickeners (for example, sodium alginate, carboxymethylcellulose sodium, sodium polyacrylate, and the like), antioxidants (for example, dibutylhydroxytoluene, butylhydroxyanisole, ascorbic acid, tocopherol, and the like), perfumes (for example, mint, strawberry, and the like), sour agents (for example, citric acid, lactose, DL-malic acid, and the like), seasoning agents (for example, DL-alanine, 5'-sodium inosinate, sodium L-glutamate, and the like), emulsifiers (for example, glycerin fatty acid ester, sucrose fatty acid ester, and the like), pH adjustors (for example, citric acid, trisodium citrate, and the like), vitamins, minerals, amino acids and coloring matters. The solid composition can be prepared according to an ordinary method. The liquid composition can be prepared in the same manner as in the liquid preparation suitable for the oral administration.

The food article of the present invention can be prepared by mixing the rice bran protein, the rice bran extract containing the rice bran protein or a solid/liquid composition containing the rice bran protein/extract into food and beverage articles. Examples of the food article include general foods such as confectionaries (for example, gum, candy, caramel, chocolate, cookie, munch, jelly, gummy candy, tablet candy, and the like), noodles (for example, buckwheat noodle, Japanese wheat noodle, Chinese noodle, and the like), dairy products (for example, milk, ice cream, yoghurt, and the like), seasoning agents (for example, miso (fermented soybean paste), soy sauce, and the like), soups and beverages (for example, juice, coffee, black tea, tea, carbonated drink, sport supplement drink, and the like), and dietary supplements (for example, nutrition supplement drink, and the like). Incidentally, the food article includes a food material too.

Ingestion of the food article containing the rice bran protein may ameliorate a lipid metabolism disorder by, for example, reducing cholesterol and neutral fat in the blood and liver, or may ameliorate hyperlipemia, obesity and type II diabetes.

It is preferred that the amount of the rice bran protein to be mixed into the composition of the present invention is usually from about 0.01 to 80% by mass in terms of the amount of protein based on the total amount of the composition.

It is preferred that the food article or the food material is labeled with the denotation on the package or the like that the product is used for amelioration of a lipid metabolism disorder, hyperlipemia, obesity or type II diabetes since it contains the rice bran protein having an excellent effect on the amelioration of a lipid metabolism disorder, hyperlipemia, obesity or type II diabetes, when the composition of the present invention is used for the food article or the food material.

The composition of the present invention can also be a health-promoting food, preferably a specified health food, which is used for amelioration of lipid metabolism disorder, hyperlipemia, obesity or type II diabetes.

The composition of the present invention can be used for amelioration of a lipid metabolism disorder, or prevention or treatment of hyperlipemia, obesity or type II diabetes, and food and beverage articles of the present invention can be used for amelioration of a lipid metabolism disorder, or suppression and amelioration of hyperlipemia, obesity or type II diabetes.

The composition of the present invention can be used for prevention or treatment of type II diabetes, particularly insulin resistant type II diabetes. The food and beverage articles of the present invention also suppress the onset of type II diabetes, particularly insulin resistant type II diabetes, and can be used for pathological amelioration or the like of the diabetes. Furthermore, the composition of the present invention prevents or treats a lipid metabolism disorder or various disorders accompanying hyperlipemia, obesity or type II diabetes, such as hypertension or arterial sclerosis (for example, cerebral arteriopathy such as cerebral infarction, and coronary artery diseases such as angina pectoris and cardiac infarction), suppresses the onset of these various disorders, or can be used for pathological amelioration of these disorders.

In the following, the present invention will be described by way of Examples and Test Examples, but the preset invention is not limited thereto.

### Example 1

30 L of water was added to 3 kg of defatted rice bran, followed by stirring at room temperature for one hour while adjusting the pH to 8.5 with an aqueous 25 W/V% sodium hydroxide solution. Then, the mixture was centrifuged (1,500 rpm x 30 seconds) to separate it into an extract and a residue. The pH of the separated extract was adjusted to 3.0 with 60 vol% sulfuric acid, followed by stirring at room temperature for one hour. Then, the precipitate was separated by centrifugation (3,000 rpm x one minute) and the acid-precipitated (separated) protein was recovered and dried by a freeze dry method. The yield of the dried matter was 230 g, or 7.7%. The protein content determined by a Kjeldahl method was 65%, and a rice bran extract containing a rice bran protein at a high concentration was obtained.

### Example 2

A rice bran extract containing a rice bran protein at a high concentration was obtained in the same manner as in Example 1, except for adding 36 vol% hydrochloric acid in place of 60 vol% sulfuric acid. The yield of the rice bran extract containing the rice bran protein was 241 g, or 8.0%. The protein content determined by a Kjeldahl method was 63%.

### Example 3

10 g of the rice bran extract containing the rice bran protein prepared in Example 1 was suspended in 60 mL of 60 vol% hydrated ethanol, followed by stirring for 5 minutes. Then, the precipitate was recovered by centrifugation (3,000 rpm x 10 minutes). The precipitate was suspended in 300 mL of 99.5% ethanol, followed by stirring for 5 minutes and further centrifugation (3,000 rpm x 10 minutes) to recover a precipitate, and the precipitate was dried by a freeze dry method. The yield of the resultant rice bran extract containing the purified rice bran protein was 8.2 g, or 82% (6.3% by defatted rice bran conversion). The protein content determined by a Kjeldahl method was 75.0%.

### Example 4

10 g of the rice bran extract containing the rice bran protein prepared in Example 1 was suspended in 100 mL of a 0.1 vol% aqueous hydrochloric acid solution, followed by stirring for 30 minutes and further centrifugation (3,000 rpm x 10 minutes) to recover a precipitate, and the precipitate was dried by a freeze dry method. The yield of the resultant rice bran extract containing the purified rice bran protein was 8.5 g, or 85% (6.5% by defatted rice bran conversion). The protein content determined by a Kjeldahl method was 69.6%.

### Example 5

10 g of the rice bran extract containing the rice bran protein prepared in Example 1 was suspended in 100 mL of 40 vol% hydrated ethanol, followed by stirring for 30 minutes and centrifugation (3,000 rpm x 10 minutes) to recover a precipitate. The precipitate was then suspended in 100 mL of a 0.1 vol% aqueous hydrochloric acid solution, followed by stirring for 30 minutes and further centrifugation (3,000 rpm x 10 minutes) to recover a precipitate. The precipitate was dried by a freeze dry method. The yield of the resultant purified rice bran protein material was 7.1 g, or 71% (5.6% by defatted rice bran conversion). The protein content determined by a Kjeldahl method was 76.4%. Test Example 1

### 1. Test Method

Four-week-old Wister male rats (CLEA Japan Inc.) were preliminarily reared for 3 days, before classified into the following 5 groups. One group is composed of 8 rats. The rats in the respective groups were fed with the respective feed shown in Table 1, which was prepared by an ordinary method. The rats could drink water freely during the experiment.

### Test Group:

Group A1: General diet (protein source: 20% by mass of casein);
Group A2: High cholesterol diet (containing 20% by mass of casein as a protein source; control group);
Group B: High cholesterol diet (containing as a protein source 10% by mass of casein and 10% by mass of the rice bran extract containing the rice bran protein obtained in Example 1);
Group C: High cholesterol diet (containing as a protein source 5% by mass of casein and 15% by mass of the rice bran extract containing the rice bran protein obtained in Example 1);
Group D: High cholesterol diet (containing as a protein source 10% by mass of casein and 10% by mass of a soy protein isolate (manufactured by Fuji Oil Co. Ltd.; protein content of 86% by mass).

**[Table 1]**

| Feed Composition | | | | | | |
|---|---|---|---|---|---|---|
| | Composition | Feed Group | | | | |
| | | Group A1 | Group A2 | Group B | Group C | Group D |
| Protein Source | Casein | 20.0% | 20.0% | 10.0% | 5.0% | 10.0% |
| | Rice Bran Extract^{a)} | - | - | 10.0% | 15.0% | - |
| | Soy Protein Isolate | - | - | - | - | 10.0% |
| Basic Feed | L-cystine | 0.3% | 0.3% | 0.3% | 0.3% | 0.3% |
| | Corn Oil | 5.0% | 5.0% | 5.0% | 5.0% | 5.0% |
| | Mineral Mixture AIN-93^{b)} | 3.5% | 3.5% | 3.5% | 3.5% | 3.5% |
| | Vitamin Mixture AIN-93^{b)} | 1.0% | 1.0% | 1.0% | 1.0% | 1.0% |
| | Cellulose | 5.0% | 5.0% | 5.0% | 5.0% | 5.0% |
| | Choline Bitartrate | 0.25% | 0.25% | 0.25% | 0.25% | 0.25% |
| | Corn Starch | 44.95% | 44.32% | 44.32% | 44.32% | 44.32% |
| | Sucrose | 20.0% | 20.0% | 20.0% | 20.0% | 20.0% |
| Cholesterol | Cholesterol | - | 0.50% | 0.50% | 0.50% | 0.50% |
| | Cholic Acid | - | 0.13% | 0.13% | 0.13% | 0.13% |
| Total | | 100% | 100% | 100% | 100% | 100% |

| | | | | | | |
|---|---|---|---|---|---|---|
| % in Table 1 represents % by mass. a) The rice bran extract containing the rice bran protein obtained in Example 1. B) Manufactured by CLEA Japan Inc. | | | | | | |

The rearing period of the animal was 21 days in all groups. During the rearing period, the body weight and intake of the feed were measured everyday. On the final day of the rearing period, the rats underwent abdominal opening surgery under ether anesthesia, and blood was collected from the subabdominal main artery. Serum was separated from the collected blood according to an ordinary method. The total cholesterol and neutral fat in the serum were respectively measured using the Cholesterol E Test Wako (manufactured by Wako Pure Chemical Industries Co.) and the Triglyceride E Test Wako (manufactured by Wako Pure Chemical Industries Co.). The resultant values were defined as blood total cholesterol and blood neutral fat. The blood adiponectin was also measured using the rat/mouse adiponection ELISA (enzyme immunoassay) kit (manufactured by Otsuka Pharmaceutical Co. Ltd.).

The liver lipid was subjected to the analysis through the following procedure: taking out the liver immediately after dissection, and a part thereof was homogenized with a tris-hydrochloric acid buffer solution (pH 7.4) according to an ordinary method.

The liver lipid was extracted by a Folch method, a part thereof was dried, and the total lipid was measured by a gravimetric method. The liver oil content was measured by a method using Soxhlet. The neutral fat content and cholesterol content in the liver were measured as follows: a constant amount of the lipid extract was dried, and 30 µL of tert-butyl alcohol and 20 µL of a mixture solution of Triton X-100: methanol (1 : 2 (V/V)) were added thereto to dissolve the dried matter, and the neutral fat and total cholesterol were measured using the Triglyceride E Test Wako (manufactured by Wako Pure Chemical Industries Co.) and the Cholesterol E Test Wako (manufactured by Wako Pure Chemical Industries Co.).

### 2. Test Result

The test results are shown in Table 2. The numerical value in the respective test groups is shown by a mean value ± a standard error. The total cholesterol and neutral fat in the blood and liver were significantly increased and a lipid metabolism disorder was present in the group A2 (control group) as compared to the group A1 (general diet group). In the groups B and C, in which the rice bran extract containing the rice bran protein was fed, the cholesterol and neutral fat in the blood and liver were significantly decreased and ameliorated as compared to those in the group A2 (control group). The adiponectin in the blood was reduced by high cholesterol diet (group A2), but the decline of the blood adiponectin was suppressed in the groups B and C, wherein the rice bran extract containing the rice bran protein was fed, and in the group D, wherein a soy protein isolate was fed.

According to the results, it is obvious that the rice bran extract containing the rice bran protein serves to ameliorate the state of a lipid metabolism disorder in the blood and liver.

As a result of a comparison between the group C and group D, it has been found that the neutral fat and total cholesterol in the blood and liver were lower in the group C than those in the group D. Consequently, it has been confirmed that the rice bran extract containing the rice bran protein is superior to the soy protein isolate in terms of the effect of ameliorating the state of a lipid metabolism disorder in the blood and liver.

**[Table 2]**

| Lipid Analysis Result in Liver and Blood in Respective Test Groups | | | | | |
|---|---|---|---|---|---|
| | Feed Group | | | | |
| | A1 | A2 | B C D | | |
| Total Cholesterol in Liver (mg/g-tissue) | 26.2 ± 10.3^{a} | 117 ± 10.7^{b} | 114.3 ± 13.3^{b} | 101.5 ± 20.2^{b} | 136.9 ± 13^{c} |
| Neutral Fat in Liver (mg/g-tissue) | 47.1 ± 15.2^{a} | 204.2 ± 71.4^{c} | 117.0 ± 30.1^{b} | 146.7 ± 41.2^{b} | 211.2 ± 30.6^{c} |
| Total Lipid in Liver (mg/g-tissue) | 32.7 ± 2.59^{a} | 134.9 ± 41.9^{c} | 95.6 ± 16.8^{b} | 110.7 ± 18.5^{b} | 113.7 ± 13.3^{bc} |
| Total Cholesterol in Blood (mg/dL) | 101.8 ± 30.3^{a} | 189.7 ± 24.6^{c} | 166.0 ± 23.4^{bc} | 130.2 ± 18.2^{ab} | 168.8 ± 13.8^{bc} |
| Neutral Fat in Blood (mg/dL) | 116.6 ± 25.3^{ab} | 138.4 ± 46.1^{b} | 94.7 ± 32.9^{a} | 114.6 ± 26.3^{ab} | 126.6 ± 26.8^{ab} |
| Adiponectin in Blood (µg/ml) | 7.1 ± 1.53^{c} | 3.7 ± 1.03^{a} | 4.3 ± 0.86^{ab} | 5.2 ± 1.11^{b} | 4.5 ± 1.02^{ab} |

The above figure denotes a mean value ± a standard error. a, b, c: There is a statistically significant difference with probability of a hazard ratio of 5% in the value attached with alphabets in the respective lines.

### Test Example 2

### 1. Test Method

The SD male rats (CLEA Japan Inc.) after ablactation were classified into 3 groups, with 6 rats in each group:
Control group: The control group under the feed groups in Table 3 (containing 20% by mass of casein as a protein source);
Rice bran protein-administered groups: The rice bran protein-administered group under the feed group in Table 3 (containing as a protein source 20% by mass of the rice bran extract containing the rice bran protein (amount of protein: 66.1% by mass) in terms of protein conversion, which is extracted in the same manner as in Example 1; and
Soybean protein-administered group: The soybean protein-administered group under the feed groups in Table 3 (containing as a protein source 20% by mass of the soy protein isolate (manufactured by Fuji Oil Co. Ltd.; protein content of 86% by mass) in terms of protein conversion). The groups were fed for 7 weeks with the respective feed shown in Table 3, which was prepared by an ordinary method. The feed was restricted to 9 g for the 1^{st} through 4^{th} days, 11 g for the 5^{th} through 7^{th} days, 15 g for the 8^{th} through 13^{th} days, 18 g for the 14^{th} through 21^{st} days, 19 g for the 22^{nd} through 27^{th} days, 20 g for the 28^{th} through 32^{nd} days. 21 g for the 33^{rd} through 35^{th} days, 22 g for the 36^{th} through 42^{nd} days and 23 g for the 43^{rd} through 49^{th} days. During the experimental period, the rats could drink water freely. The body weight, fecal weight (feces of 48 hours), nitrogen content in the feces and nitrogen content in the urine were measured diachronically. The nitrogen contents in the feces and urine were measured by a Kjehdahl method.
After 7 weeks, the rats underwent abdominal opening surgery under ether anesthesia to collect blood from the subabdominal main artery, and were killed by bleeding. After the rats were brought into death by bleeding, the livers, soleus muscles, visceral fat tissues (epididymal fat tissues and perirenal fat tissues) and appendixes were extracted, and the weight of the respective tissues was weighed.

The blood total cholesterol, blood neutral fat, liver total cholesterol and liver neutral fat were measured in the same manner as in Test Example 1. The liver total lipid was calculated from the lipid weight obtained by extraction through a Folch method.

**[Table 3]**

| | Composition | Feed Groups | | |
|---|---|---|---|---|
| | | Control Group | Rice Bran Protein-administered Group | Soybean Protein-administered Group |
| Protein | Casein | 20.0% | - | - |
| | Rice Bran Extract^{a}) | - | 25.36% | - |
| | Soy Protein Isolate | - | - | 20.89% |
| Basic Feed | L-cystine | 0.3% | 0.3% | 0.3% |
| | Corn Oil | 10.0% | 10.0% | 10.0% |
| | Mineral Mixture AIN-93^{b)} | 3.5% | 3.5% | 3.5% |
| | Vitamin Mixture AIN-93^{b)} | 1.0% | 1.0% | 1.0% |
| | Cellulose | 5.0% | 5.0% | 5.0% |
| | Choline Bitartrate | 0.25% | 0.25% | 0.25% |
| | Corn Starch | 39.95% | 36.38% | 39.36% |
| | Sucrose | 20.00% | 18.21% | 19.70% |
| Total | | 100% | 100% | 100% |

| | | | | |
|---|---|---|---|---|
| % in Table 3 represents % by mass. a) The rice bran extract containing the rice bran protein obtained in the same manner as in Example 1 (Protein Mass: 66.1% by mass). b) Manufactured by CLEA Japan Inc. | | | | |

### 2. Test Result

(1) The growth increment, weight ratio of each tissue and blood/liver lipid after rearing for 7 weeks are shown in Table 4. It was recognized that the rice bran protein-administered group and the soybean protein-administered group were more effective than the control group in terms of decreasing any of the weight ratios of an epididymal fat tissue, a perirenal fat tissue and an interperitoneal fat tissue. The effect was greater in the rice bran protein-administered group than in the soybean protein-administered group. When the epididymal fat tissue and the perirenal fat tissue were observed with an electron microscope, it was found that the fat cells of both the epididymal fat tissue and perirenal fat tissue were smaller in the rice bran protein-administered group. This indicates that the rice bran extract containing the rice bran protein is superior to the soy protein isolate in the effect of reducing the visceral fat. An increase in the weight ratio of appendix and a decrease in the pH in the content of the appendix were also found greater in the rice bran protein-administered group and the soybean protein-administered group, as compared to those of the control group. The increase in the weight ratio of the appendix and the decrease level of the pH in the content of the appendix were significantly greater in the rice bran protein-administered group than in the soybean protein-administered group. There was also a tendency that the weight ratio of the soleus muscle was higher in the rice bran protein-administered group. The results indicate that the rice bran extract containing the rice bran protein is superior to the soy protein isolate in the effect on reduction of the visceral fat and, conversely, increase of the muscle. The increase of undigested food suppresses the absorption of glucide and lipid in the intestine and thus helps to prevent obesity, and also suppresses the elevation of blood sugar after meal, resulting in possible suppression of insulin secretion. The inventors actually measured the lipid content in the feces and confirmed that the lipid content in the feces was higher in the rice bran protein-administered group.
The blood total cholesterol, liver total lipid, liver total cholesterol and liver neutral fat significantly declined in the rice bran protein-administered group, as compared to the control group.

**[Table 4]**

| | Control Group | Rice Bran Protein-administered Group | Soybean Protein-administered Group |
|---|---|---|---|
| Growth Increment (g)¹⁾ | 323 ± 5^{b} | 329 ± 4^{b} | 310 ± 4^{a} |
| Dietary Efficiency (%)²⁾ | 0.357 ± 0.006^{b} | 0.364 ± 0.004^{b} | 0.343 ± 0.004^{a} |
| Weight Ratio of Tissue (g/100g weight) | | | |
| Liver | 3.60 ± 0.18 | 3.60 ± 0.11 | 3.86 ± 0.23 |
| Soleus Muscle | 1.34 ± 0.08 | 1.43 ± 0.02 | 1.33 ± 0.05 |
| Appendix | 0.681 ± 0.083^{ab} | 0.978 ± 0.042^{b} | 0.873 ± 0.067^{b} |
| Periepididymal Fat | 1.19 ± 0.04^{b} | 1.04 ± 0.03^{a} | 1.23 ± 0.07^{b} |
| Perirenal Fat | 1.74 ± 0.09^{ab} | 1.52 ± 0.12^{a} | 2.09 ± 0.24^{b} |
| Interperitoneal Fat | 2.93 ± 0.083^{ab} | 2.56 ± 0.14° | 3.32 ± 0.29^{b} |
| Blood lipid (mg/100 mL) | | | |
| Total Cholesterol | 78.2 ± 4.5^{b} | 57.8 ± 4.7^{a} | 52.5 ± 3.5^{a} |
| Neutral Fat | 61.1 ± 6.5 | 88.7 ± 7.6 | 74.6 ± 7.1 |
| Liver lipid | | | |
| (mg/g) | | | |
| Total lipid | 117.7 ± 8.9^{b} | 94.6 ± 5.5^{a} | 118.4 ± 6.7^{b} |
| Total Cholesterol | 7.01 ± 0.25^{b} | 6.28 ± 0.13^{a} | 7.01 ± 0.22^{b} |
| Neutral Fat | 43.1 ± 3.6^{b} | 32.5 = 3.0^{a} | 50.3 ± 4.2^{b} |

| | | | |
|---|---|---|---|
| The above value denotes a mean value ± a standard error. 1) Growth increment: weight after 7-week rearing - weight at start of test 2) Dietary efficiency: growth increment during rearing period (g)/dietary intake during rearing period 3) Interperitoneal fat: periepididymal fat + perirenal fat a, b, c: There is a statistically significant difference with probability of a hazard ratio of 5% in the value attached with alphabets in the respective lines. | | | |

(2) The fecal amount, nitrogen content in the urine, and the digestibility and biological value of protein are shown in Table 5. The fecal amount and nitrogen contents in the feces and urine significantly increased in the rice bran protein-administered group and the soybean protein-administered group, as compared to the control group. This corresponds to the general finding that a plant protein (for example, rice bran protein and soybean protein) is inferior to an animal protein (for example, casein) in terms of protein quality indicated by a digestibility and a biological value. In the comparison between the rice bran extract containing the rice bran protein and the soy protein isolate, the rice bran extract containing the rice bran protein had a lower digestibility and a higher biological value. The result indicates that the rice bran extract containing the rice bran protein has resistance particularly against a digestive enzyme and functions like a kind of dietary fiber, while it is an excellent protein with high quality. That is, the rice bran protein is a particularly useful protein among plant proteins, which is capable of controlling the energy intake without damaging a nutritional condition in the dietary habit that leads to a lipid metabolism disorder as a result of excessive nutrition.

**[Table 5]**

| | Control Group | Rice Bran Protein-administered Group | Soybean Protein-administered Group |
|---|---|---|---|
| Dry Fecal Weight (g/2 days) | | | |
| 10 to 11 days after start of test | 2.07 ± 0.15^{a} | 2.98 ± 0.19^{b} | 0.28 ± 0.08^{a} |
| 45 to 46 days after start of test | 3.26 ± 0.13^{a} | 4.07 ± 0.28^{b} | 3.40 ± 0.20^{a} |
| Nitrogen Content in Feces (mg/2 days) | | | |
| 10 to 11 days after start of test | 33.4 ± 3.8^{a} | 79.6 ± 6.6^{c} | 51.0 ± 2.8^{b} |
| 45 to 46 days after start of test | 62.4 ± 2.4^{a} | 126.2 ± 8.6^{c} | 88.8 ± 5.6^{b} |
| Nitrogen Content in Urine (mg/2 days) | | | |
| 10 to 11 days after start of test | 218 ± 12^{a} | 258 ± 6^{b} | 290 ± 6^{c} |
| Protein Digestibility (%) | | | |
| 10 to 11 days after start of test | 95.9 ± 0.5^{c} | 90.5 ± 0.8^{a} | 93.4 ± 0.4^{b} |
| 45 to 46 days after start of test | 95.0 ± 0.2^{c} | 89.7 ± 0.5^{a} | 92.3 ± 0.9^{b} |
| Biological Value (%) | | | |
| 10 to 11 days after start of test | 72.2 ± 1.6^{c} | 66.2 ± 0.9^{b} | 59.8 ± 1.1^{a} |

| | | | |
|---|---|---|---|
| The above value denotes a mean value ± a standard error. 1) Protein digestibility: (nitrogen intake - nitrogen content in feces)/nitrogen intake x 100 2) Biological value: (nitrogen intake - nitrogen content in feces - nitrogen content in urine)/(nitrogen intake - nitrogen content in feces) x 100 a, b, c: There is a statistically significant difference with probability of a hazard ratio of 5% in the value attached with alphabets in the respective lines. | | | |

### Test Example 3

### 1. Test Method

The test was conducted in the same manner as in Test Example 1, except for classifying the test groups as follows, and the serum was separated and the liver was extracted. The total cholesterol and neutral fat in the serum and liver, and GOT (gulutamic-oxaloacetic transaminase), GPT (gulutamic-pyruvic transaminase) and leptin in the serum were measured. The measurement of the total cholesterol and neutral fat was conducted in the same manner as in Example 1. GOT and GPT were measured by using the Transaminase CII-Test Wako (manufactured by Wako Pure Chemical Industries Co.). Leptin was measured by using the Morinaga Rat Leptin Measurement Kit (manufactured by Morinaga Institute of Biological Science Inc.).

### Test Group:

Group A: High cholesterol diet (identical to the group A2 in Table 1: control group);
Group B: High cholesterol diet (same as the group B in Table 1, except for replacing the rice bran extract with the rice bran extract containing the rice bran protein (protein content: 75% by mass) extracted in the same manner as in Example 1 in an amount of 10% by mass in terms of protein conversion);
Group C: High cholesterol diet (same as the group B in Table 1, except for replacing the rice bran extract with the rice bran extract containing the rice bran protein (protein content: 65% by mass) extracted in the same manner as in Example 1 in an amount of 10% by mass in terms of protein conversion);
Group D: High cholesterol diet (identical to the group D in Table 1).

### 2. Test Result

The results are shown in Table 6. Corresponding to the results of Test Example 1, the present results show that the cholesterol in the serum and the lipid content in the liver of the cholesterol-ingesting rats were suppressed by the rice bran extract containing the rice bran protein. Furthermore, the serum GOT and GPT activities as the indicators of liver damage were suppressed by the rice bran extract containing the rice bran protein. As a result, it is expected that the rice bran extract containing the rice bran protein improves the liver function, and prevents/ameliorates a lipid metabolism disorder, when lipid accumulates in the liver and contributes to decreasing the liver function.

**[Table 6]**

| | Feed Groups | | | |
|---|---|---|---|---|
| | A | B | C | D |
| Liver Total Cholesterol (mg/g-tissue) | 96.6 ± 11.9^{b} | 69.5 ± 9.0^{a} | 65.2 ± 20.4^{a} | 75.7 ± 8.5^{a} |
| Liver Neutral Fat (mg/g-tissue) | 137.6 ± 22.4^{b} | 110.9 ± 16.8^{a} | 104.9 ± 18.5^{a} | 98.2 ± 39.1^{a} |
| Blood Total Cholesterol (mg/dL) | 168.7 ± 16.4^{c} | 125.8 ± 21.6^{a} | 112.5 ± 13.4^{a} | 147.0 ± 11.1^{b} |
| Blood Neutral Fat (mg/dL) | 140.2 ± 23.5 | 120.1 ± 14.3 | 137.4 ± 28.2 | 121.4 ± 41.3 |
| Blood GOT (ku/dL) | 103.1 ± 31.6^{b} | 88.2 ± 27.8^{ab} | 77.2 ± 15.3^{a} | 92.0 ± 28.7^{ab} |
| Blood GPT (ku/dL) | 24.3 ± 5.3^{b} | 21.2 ± 5.5^{ab} | 19.5 ± 3.3^{a} | 19.1 ± 3.9^{a} |
| Blood Leptin (ng/mL) | 1.46 ± 4.9 | 1.26 ± 4.9 | 1.17 ± 1.7 | 1.80 ± 1.2 |

| | | | | |
|---|---|---|---|---|
| The above value denotes a mean value ± a standard error. a, b, c: There is a statistically significant difference with probability of a hazard ratio of 5% in the value attached with alphabets in the respective lines. | | | | |

### INDUSTRIAL APPLICABILITY

The composition for amelioration of a lipid metabolism disorder of the present invention is useful for prevention, treatment or amelioration of obesity and/or type II diabetes, and food and beverage articles of the present invention are useful as a health-promoting food which prevents or ameliorates obesity and/or type II diabetes.

## Claims

1. A composition for amelioration of a lipid metabolism disorder, which comprises a rice bran protein.

2. The composition according to claim 1, wherein the rice bran protein is a protein contained in rice bran extract.

3. The composition according to claim 2, wherein the amount of the rice bran protein relative to the rice bran extract is not less than 50°s by mass.

4. The composition according to any one of claims 1 to 3, wherein the lipid metabolism disorder is caused by at least one disease selected from hyperlipemia, obesity and type II diabetes.

5. The composition according to any one of claims 1 to 4, which is a medicine.

6. The composition according to any one of claims 1 to 5, which is a food article or a food material.

7. A method for amelioration of a lipid metabolism disorder, which comprises administering a composition containing a rice bran protein to a mammal including a human.

8. The method according to claim 7, wherein the rice bran protein is a protein contained in rice bran extract.

9. The method according to claim 8, wherein the amount of the rice bran protein relative to the rice bran extract is not less than 50% by mass.

10. The method according to any one of claims 7 to 9, wherein the lipid metabolism disorder is caused by at least one disease selected from hyperlipemia, obesity and type II diabetes.

11. Use of a rice bran protein for producing a medicine, a food article or a food material for amelioration of a lipid metabolism disorder.

12. The use according to claim 11, wherein the rice brain protein is a protein contained in rice bran extract.

13. The use according to claim 12, wherein the amount of the rice bran protein relative to the rice bran extract is not less than 50% by mass.

14. The use according to any one of claims 11 to 13, wherein the lipid metabolism disorder is caused by at least one disease selected from hyperlipemia, obesity and type II diabetes.
